# EUROPÄISCHE PATENTSCHRIFT

(11) **EP 0 703 452 B1**
(45) Veröffentlichungstag und Bekanntmachung des Hinweises auf die Patenterteilung: **02.05.2003**
(21) Anmeldenummer: 95114973.1
(22) Anmeldetag: 22.09.1995
(51) Int. Cl.: G01N 33/543, G01N 33/53, G01N 33/573

(54) **Verfahren zum qualitativen und/oder quantitativen Nachweis einer zu bestimmenden Substanz**
Method for qualitative and/or quantitative determination of a compound
Méthode qualitative et/ou quantitative pour déterminer une substance

(30) Priorität: 23.09.1994 DE 4434093
(43) Veröffentlichungstag der Anmeldung: 27.03.1996
(73) Patentinhaber: Roche Diagnostics GmbH, 68305 Mannheim (DE)
(72) Erfinder: Naser, Werner, Dr., D-82377 Penzberg (DE)
(74) Vertreter: Böhm, Brigitte, Dipl.-Chem. Dr.

(56) Entgegenhaltungen:
- EP-A- 0 265 244
- EP-A- 0 296 544
- EP-A- 0 331 127
- WO-A-91/12336
- WO-A-94/10573
- WO-A-95/23801
- FR-A- 2 410 278

## Beschreibung

Die vorliegende Erfindung betrifft ein Verfahren zum qualitativen oder/und quantitativen Nachweis einer zu bestimmenden Substanz in einer Testprobe mit Hilfe eines Immuno- oder Nukleinsäure-Hybridisierungsassays, eine inaktivierte Festphase zur Verwendung in einem solchen Assay sowie die Verwendung dieser inaktivierten Festphase zur Entstörung von Immuno- oder Nukleinsäure-Hybridisierungsassays durch Abfangen von unspezifisch bindenden Komponenten in einer zu analysierenden Testprobe. Schließlich betrifft die Erfindung auch noch Testkits, enthaltend die üblichen Komponenten eines Immuno-oder Nukleinsäure-Hybridisierungsassays sowie eine erfindungsgemäße inaktivierte Festphase.

Immuno- und Nukleinsäure-Hybridisierungsassays dienen heutzutage dem schnellen und einfachen Nachweis von Substanzen, beispielsweise auf diagnostischem Gebiet. Mit ihnen kann die Gegenwart von körpereigenen oder körperfremden Substanzen in biologischen Proben schnell und zuverlässig nachgewiesen werden. Immuno- und Nukleinsäure-Hybridisierungsassays basieren auf dem Prinzip, daß eine nachzuweisende Substanz mit einer anderen Substanz spezifisch bindet, wobei bei geeigneter Ausgestaltung des Assays mit Hilfe dieser anderen Substanz die Anwesenheit der festzustellenden Substanz nachgewiesen werden kann. Die spezifische Bindungsfähigkeit der zu bestimmenden Substanz mit einer oder mehreren weiteren Substanzen wird also ausgenutzt, um den Nachweis zu ermöglichen. Hierbei können jedoch auch Nebenreaktionen auftreten, wie unerwünschte Wechselwirkungen und unspezifische Bindereaktionen. Insbesondere bei Verwendung einer Festphase, an die spezifisch bindende Substanzen immobilisiert oder immobilisierbar sind, tritt das Problem auf, daß oftmals in der Testprobe zusätzliche Substanzen enthalten sind, die ebenfalls an diese Festphase binden und dabei falsch positive Signale verursachen. Es kann hierdurch das Hintergrundsignal erhöht werden, ebenso kann eine stärkere Streuung der Signale erfolgen und damit eine verringerte Sensitivität und Spezifität des betreffenden Tests die Folge sein.

Dokument FR-A-2 410 278 offenbart beispielsweise einen heterogenen Immunoassay, der zur Verminderung des Einflusses unspezifisch bindender Komponenten die Verwendung von zwei Festphasen beinhaltet. An die erste Festphase wird ein für den zu bestimmenden Antikörper spezifisches Antigen gekoppelt. Die zweite Festphase dient zur Bindung anderer Proteine, wobei diese Festphase kein für den zu bestimmenden Antikörper spezifisches Antigen enthält.

EP-A-0 331 127 beschreibt die Bindung eines Fangreagenzes an eine Festphase über ein spezifisches Bindungspaar, welches z.B. aus spezifisch aneinander bindenden Polymeren besteht.

In Dokument EP-A-0 265 244 wird eine sequenzielle Inkubation von Festphasen in einem Assay beschrieben. Es wird die aufeinanderfolgende Zugabe von zwei Arten von Partikeln, die mit Nukleinsäuren markiert sind, welche spezifisch für unterschiedliche Fangreagenzien sind, offenbart.

Zur Vermeidung von Wechselwirkungen mit nicht spezifischen Faktoren wird in der europäischen Patentanmeldung EP 0 163 312 vorgeschlagen, den Reagenzien des Immunoassays ultrafeine Partikel zuzusetzen mit einer maximalen Durchschnittsgröße von 0,2 µm, welche so ausgebildet sind, dass sie mit der die unspezifische Reaktion verursachenden Substanz bindefähig sind und diese abfangen. Hierzu ist jedoch eine spezielle Vorbereitung dieser ultrafeinen Partikel nötig, auch muss bekannt sein, welche unspezifischen Faktoren in der Probe tatsächlich vorhanden sind.

Es wäre jedoch von Vorteil, wenn eine Methode zur Verfügung stünde, ganz generell und allgemein Störungen in Immuno- oder Nukleinsäure-Hybridisierungsassays zu beseitigen, auch ohne Kenntnis der tatsächlich vorhandenen Störsubstanzen. Es war daher Aufgabe der vorliegenden Erfindung, ein entsprechendes Verfahren bereitzustellen. Diese Aufgabe wird erfindungsgemäß gelöst durch ein Verfahren zum qualitativen oder/und quantitativen Nachweis einer zu bestimmenden Substanz in einer Testprobe mit Hilfe eines Immuno-oder Nukleinsäure-Hybridisierungsassays, bei dem die folgenden Komponenten eingesetzt werden:
a) ein Fangreagenz, das mittels zweier verschiedener Bindungsstellen 1) gegebenenfalls zusammen mit weiteren Testkomponenten einen spezifischen Nachweis der zu bestimmenden Substanz ermöglicht und 2) über ein spezifisches Bindungspaar, dessen einer Partner mit dem Fangreagenz verbunden ist und dessen zweiter Partner an eine aktive Festphase gekoppelt ist, an eine aktive Festphase gebunden oder bindefähig ist,
b) eine aktive Festphase an die der zweite Partner des spezifischen Bindungspaares gekoppelt ist, und
c) eine inaktivierte Festphase, die der aktiven Festphase entspricht, die jedoch so modifiziert ist, dass das Fangreagenz nicht daran binden kann,
wobei die Testprobe entweder zuerst mit der inaktivierten Festphase alleine und erst später mit der aktiven Festphase oder gleichzeitig mit aktiver und inaktivierter Festphase in Kontakt gebracht wird, wobei das Fangreagenz und gegebenenfalls weitere Testkomponenten entweder von Anfang an anwesend sind oder aber erst später zugegeben werden, und ein Nachweis der zu bestimmenden Substanz erfolgt.

Das erfindungsgemäße Fangreagenz ist so ausgestaltet, daß es zwei Arten von Bindungsstellen aufweist. Mittels einer Art von Bindungsstelle kann es mit einem Partner eines spezifischen Bindungspaares koppeln. Auch die Kopplung an mehrere Partner von Bindungspaaren ist möglich. Weiterhin weist das erfindungsgemäße Fangreagenz eine weitere Art von Bindungsstelle auf, die einen spezifischen Nachweis der zu bestimmenden Substanz ermöglicht. Bevorzugt sind diese Bindungsstellen mit der zu bestimmenden Substanz oder mit einem Komplex aus der zu bestimmenden Substanz und weiteren Testkomponenten spezifisch bindefähig. Ein Fangreagenz kann auch mehrere Bindungsstellen dieser Art aufweisen, beispielsweise weisen Antikörper zwei Epitope mit spezifischen Bindungsstellen auf.

Im erfindungsgemäßen Verfahren werden als Fangreagenz bevorzugt Substanzen eingesetzt, die mit der zu bestimmenden Substanz spezifisch bindefähig sind. Handelt es sich bei der zu bestimmenden Substanz um einen Antikörper, so wird als Fangreagenz entweder ein entsprechendes Antigen, ein Hapten oder aber ein gegen den zu bestimmenden Antikörper gerichteter zweiter Antikörper oder ein Fragment davon verwendet. Handelt es sich bei der zu bestimmenden Substanz dagegen um ein Antigen, wird als Fangreagenz ein Antikörper oder Antikörperfragment verwendet. Handelt es sich bei der zu bestimmenden Substanz um eine Nukleinsäure, wird als Fangreagenz ein Nukleinsäureoligomer oder -polymer oder ein Nukleinsäureanalogon verwendet. Bevorzugt wird dabei als Nukleinsäureanalogon eine peptidische Nukleinsäure verwendet.

Das Fangreagenz ist dabei erfindungsgemäß so ausgestaltet, daß es mit einer mit der Bindung der zu bestimmenden Substanz nicht interferierenden Stelle des Moleküls über ein spezifisches Bindungspaar an eine Festphase gebunden werden kann bzw. bereits an die Festphase gebunden vorliegt. Diese Bindung kann entweder direkt über das Bindungspaar erfolgen. Die Kopplung des Fangreagenzes an die Festphase kann jedoch auch über ein Bindungssystem, bestehend aus mehr als zwei miteinander bindefähigen Komponenten bestehen. Grundsätzlich ist ein Partner des spezifischen Bindungspaares mit dem Fangreagenz verbunden und ein zweiter Partner des spezifischen Bindungspaares ist an die Festphase gekoppelt. Diese Kopplung kann wiederum nach allen dem Fachmann geläufigen Methoden erfolgen, und die Kopplung des Fangreagenzes an die Festphase über das spezifische Bindungspaar kann vor Durchführung des Immunoassays erfolgen oder aber auch erst während des Immunoassays erfolgen, indem erst zu einem späteren Zeitpunkt das Fangreagenz zu den anderen Komponenten des Immunoassays zugegeben wird.

In einer bevorzugten Ausführungsform der Erfindung verwendet man als spezifisches Bindepaar Biotin/Avidin-, Biotin/Streptavidin-, Antigen/Antikörper-, Hapten/Antikörper-Paare bzw. miteinander spezifisch bindefähige Fragmente hiervon. In einer besonders bevorzugten Ausführungsform der Erfindung verwendet man als spezifisches Bindepaar Biotin/Avidin bzw. Biotin/Streptavidin.

Der Nachweis der zu bestimmenden Substanz erfolgt erfindungsgemäß bevorzugt über ein Nachweisreagenz, das zusammen mit dem Fangreagenz einen spezifischen Nachweis der zu bestimmenden Substanz ermöglicht und eine indirekte oder direkte Markierung trägt. Mit direkt markierten Nachweisreagenzien erfolgt der Nachweis dadurch, daß das Nachweisreagenz entweder an die zu bestimmende Substanz bindet und einen detektierbaren Sandwichkomplex bildet, der die Markierung trägt, oder kompetitiv zu der zu bestimmenden Substanz an das Fangreagenz bindet und einen Nachweisreagenz-Fangreagenz-Komplex bildet, der die Markierung trägt. Nachweisreagenzien mit indirekter Markierung umfassen mehrere Komponenten, wobei die Komponente, die an die zu bestimmende Substanz bzw. an das Fangreagenz bindet, unmarkiert ist und mit einer weiteren Komponente, die die Markierung trägt, bindefähig ist.

Geeignete Markierungen sind dem Fachmann bekannt. Bevorzugt werden hierzu radioaktive Markierungen, Chemilumineszenz-, Fluoreszenz- oder Elektrochemilumineszenzmarker, gefärbte Partikel wie z.B. Metallsolpartikel oder gefärbter oder ungefärbter Latex verwendet. Die Markierung kann auch ein indirektes Signal liefern, wie beispielsweise bei Enzymmarkierungen mit Enzymen wie Peroxidase, β-Galactosidase oder alkalischer Phosphatase. Die Bestimmung der Markierung kann erfindungsgemäß bevorzugt an der aktiven Festphase oder/und im Probenüberstand erfolgen.

Zum Nachweis der zu bestimmenden Substanz können erfindungsgemäß alle bekannten Nachweisverfahren verwendet werden. Der Nachweis kann beispielsweise als sogenanntes Verdrängungsverfahren oder als Sandwich-Verfahren durchgeführt werden. Beim erfindungsgemäß bevorzugten Verdrängungsverfahren wird ein markiertes Nachweisreagenz eingesetzt, das die gleiche oder eine ähnliche Bindungsstelle wie die zu bestimmende Substanz aufweist und somit mit der zu bestimmenden Substanz konkurriert. Handelt es sich bei der zu bestimmenden Substanz beispielsweise um einen Antikörper, so werden zum Nachweis dieses Antikörpers im Verdrängungsverfahren als Fangreagenz ein entsprechendes Antigen und ein markierter Antikörper zugegeben, wobei der markierte Antikörper die gleiche oder eine ähnliche Bindungsspezifität wie der zu bestimmende Antikörper aufweist. Das Antigen kann direkt über ein spezifisches Bindungspaar, dessen einer Partner mit dem Antigen verbunden ist und dessen zweiter Partner an die aktive Festphase gekoppelt ist, an die aktive Festphase gebunden oder mit dieser bindefähig sein. Das Antigen kann auch über einen an die Festphase bindefähigen oder gebundenen zweiten Antikörper indirekt an die Festphase immobilisiert werden oder sein. Dieser zweite Antikörper kann ebenfalls wie der markierte Antikörper die gleiche oder eine ähnliche Spezifität wie der zu bestimmende Antikörper aufweisen. Der Probe-Antikörper konkurriert mit dem markierten Antikörper und gegebenenfalls mit dem zweiten Antikörper, um die Bindung an das Antigen.

Handelt es sich bei der zu bestimmenden Substanz um ein Antigen, so wird zum Nachweis im Verdrängungsverfahren bevorzugt ein immunologisch konkurrierendes Analogon des zu bestimmenden Antigens verwendet. Beispielsweise handelt es sich dabei um das gleiche Antigen oder/und ein Antigen mit ähnlicher Bindungsspezifizität. Handelt es sich um eine Nukleinsäure, so wird als Nachweisreagenz bevorzugt ein Nukleinsäureoligomer oder -polymer oder ein Nukleinsäureanalogon und besonders bevorzugt eine peptidische Nukleinsäure verwendet.

Erfindungsgemäß besonders bevorzugt wird der Nachweis der zu bestimmenden Substanz im Sandwich-Verfahren durchgeführt. Das Nachweisreagenz ist dabei mit der zu bestimmenden Substanz spezifisch bindefähig. Handelt es sich bei der zu bestimmenden Substanz wiederum um einen Antikörper, so kann als Nachweisreagenz entweder ein Antigen, ein Hapten oder ein gegen den ersten Antikörper gerichteter zweiter Antikörper oder ein Antikörperfragment verwendet werden. Handelt es sich bei der zu bestimmenden Substanz dagegen um ein Antigen, so wird ein Antikörper oder ein Antikörperfragment als Nachweisreagenz verwendet, welches an ein anderes Epitop des Antigens bindet als das Fangreagenz. Handelt es sich bei der zu bestimmenden Substanz um eine Nukleinsäure wie beispielsweise ein Oligonukleotid oder um einen DNA- oder RNA-Abschnitt, wird als Fangreagenz geeigneterweise ein Nukleinsäureoligomer oder -polymer oder eine analoge Struktur mit einem zu der zu bestimmenden Substanz komplementären Sequenzbereich verwendet.

Als Nukleinsäureanaloga können Strukturen mit modifizierten Nukleobasen, Zuckern oder/und internukleotidischen Bindungen verwendet werden. Besonders gut geeignete Nukleotidanaloga sind peptidische Nukleinsäuren/PNA. Generell sind die für derartige Immuno- und Nukleinsäure-Hybridisierungsassays und insbesondere für "Sandwich-Assays" geeigneten Fang- und Nachweisreagenzien dem Fachmann bekannt und müssen hier nicht abschließend erläutert werden.

Im erfindungsgemäß bevorzugten Sandwich-Nachweis-Verfahren wird also ein Komplex gebildet aus einer zu bestimmenden Substanz, einem Fangreagenz, das die Bindung an eine Festphase vermittelt und einem Nachweisreagenz, das eine Markierung trägt und damit den Nachweis der Anwesenheit der zu bestimmenden Substanz ermöglicht. Die Reihenfolge der Zugabe der einzelnen Komponenten des Tests ist bei der Testdurchführung unkritisch.

Neben Fangreagenz und gegebenenfalls Nachweisreagenz wird für das erfindungsgemäße Verfahren eine aktive Festphase verwendet. Diese aktive Festphase enthält entweder das Fangreagenz bereits in gebundener Form oder ist derart ausgestaltet, daß das Fangreagenz an die feste Phase binden kann. Der Ausdruck "aktive" Festphase soll verdeutlichen, daß diese Festphase über das Fangreagenz den Nachweis der zu bestimmenden Substanz ermöglicht. Bevorzugt wird über das Fangreagenz die Bindung des Nachweisreagenzes an die aktive Festphase ermöglicht, wodurch eine Separation von ungebundenen Komponenten erfolgen und der Nachweis der zu bestimmenden Substanz geführt werden kann. Im erfindungsgemäßen Verfahren wird jedoch im Gegensatz zu den bisher bekannten Immuno- und Nukleinsäure-Hybridisierungsassays noch eine weitere, inaktive Festphase verwendet, die zwar weitgehend der aktiven Festphase entspricht, jedoch so modifiziert ist, daß das Fangreagenz nicht daran binden kann. An diese inaktive Festphase können jedoch unspezifische Bindungen, die nicht durch das Fangreagenz bzw. dessen spezifische Bindung an die Festphase vermittelt werden, erfolgen. Die Verwendung dieser inaktiven Festphase, welche entweder mit der Testprobe allein vorinkubiert werden kann, die jedoch auch in Kombination mit einem oder mehreren oder schließlich allen anderen Komponenten des Immunoassays eingesetzt werden kann, ermöglicht es, unspezifische Bindungen abzufangen und damit zu verhindern, daß an der aktiven Festphase durch unspezifische Bindungen eine wie auch immer geartete Überbrückung erfolgt, so daß ein Nachweisreagenz an die aktive Festphase gebunden wird und so ein falsch positives Signal erzeugt wird.

Bevorzugt wird die Testprobe zuerst mit der inaktiven Festphase alleine inkubiert. Dabei können alle mit der Festphase bindefähigen unspezifischen Substanzen abreagieren, wonach die Testprobe von der inaktiven Festphase abgetrennt und dann mit der aktiven Festphase und den anderen Komponenten, wie Fangreagenz und gegebenenfalls Nachweisreagenz und weiteren Testkomponenten, in beliebiger Reihenfolge oder Kombination inkubiert wird, und über das Markierungssignal der Nachweis der zu bestimmenden Substanz geführt werden kann.

In einer anderen bevorzugten Ausführungsform werden die inaktive und die aktive Festphase gleichzeitig mit der Testprobe und gegebenenfalls auch den anderen Komponenten des Immuno- oder Nukleinsäure-Hybridisierungsassays in Kontakt gebracht, was ebenfalls durch Bindung unspezifischer Substanzen auch an die inaktive Festphase die Hintergrund- und Störsignale doch zumindest erheblich reduziert.

Als Festphasen können im Rahmen der Erfindung alle für Immunoassays verwendbare Festphasen eingesetzt werden. Bevorzugt werden als Festphase Plastikröhrchen, Mikrotiterplatten, Glasoder Plastikbeads oder Latexpartikel verwendet. Es ist jedoch auch die Verwendung von Teststreifen aus Papier oder Plastik möglich.

Die Inaktivierung der Festphase kann in verschiedener Weise erfolgen. Es muß hierbei jedoch sichergestellt sein, daß die spezifische Bindung des Fangreagenzes an die Festphase nicht stattfinden kann. In einer besonders bevorzugten Ausführungsform der Erfindung, in der als spezifisches Bindungspaar Biotin/Streptavidin oder Biotin/Avidin verwendet wird, und davon Avidin bzw. Streptavidin an die Festphase gebunden vorliegen, bewirkt man bei der Herstellung der inaktiven Festphase eine Inaktivierung von Avidin oder Streptavidin durch Absättigung mit Biotin oder einem Biotinderviat. Desweiteren kann bevorzugterweise die Inaktivierung von Avidin oder Streptavidin, welche an die Festphase gebunden sind oder daran gebunden werden sollen, durch kovalente Modifizierung des aktiven Zentrums erfolgen. Eine solche kovalente Modifizierung wird vorzugsweise durch eine Derivatisierung mindestens einer Aminosäure des aktiven Zentrums oder durch eine kovalente Kopplung von Biotin an das aktive Zentrum bewirkt. Die kovalente Kopplung kann hierbei bevorzugt erfolgen, indem photoaktivierbares Biotin, wie beispielsweise Biotin-DADOO-AB an das Avidin oder Streptavidin gekoppelt wird.

Es kann jedoch auch - und dies ist ebenfalls im Rahmen der Erfindung besonders bevorzugt - gentechnologisch hergestelltes Avidin oder Streptavidin zur Herstellung der inaktiven Festphase verwendet werden, bei dem durch gentechnologische Veränderung des aktiven Zentrums, wie Substitution, Deletion oder Insertion einzelner oder mehrerer Aminosäuren die Bindung von Biotin verhindert wird.

Auch die Verwendung von Fragmenten von Avidin oder Streptavidin erscheint möglich, solange die damit gekoppelte Festphase noch ausreichend unspezifische Bindung abfangen kann. Dies kann durch einfache Vorversuche festgestellt werden.

Als Testprobe können allgemein wäßrige Proben eingesetzt werden, wobei Körperflüssigkeiten und insbesondere Blut, Blutplasma, Serum, Speichel, Gewebsflüssigkeit, wie beispielsweise durch die Haut mit Hilfe von "skin-patches" gewonnene Gewebsflüssigkeit, Liquor oder Urin in der Diagnostik ihre Anwendung finden. Bevorzugt ist daher die Testprobe eine solche Körperflüssigkeit.

Im erfindungsgemäßen Verfahren ist es bevorzugt, für den Fall, daß es sich bei der zu bestimmenden Substanz um ein Antigen handelt, als Fang- und gegebenenfalls Nachweisreagenz Antikörper oder Antikörperfragmente zu verwenden. Für den umgekehrten Fall, daß es sich bei der nachzuweisenden Substanz um einen Antikörper handelt, verwendet man als Fang- oder/und Nachweisreagenzien Antigene oder/und Haptene, wobei eines der beiden Reagenzien ein Antigen oder/und ein Hapten sein kann und das andere Reagenz ein gegen den zu bestimmenden Antikörper gerichteter zweiter Antikörper ist. Handelt es sich bei der zu bestimmenden Substanz um eine Nukleinsäure wie beispielsweise ein Oligonukleotid oder um einen DNA- oder RNA-Abschnitt, wird als Fangreagenz geeigneterweise ein Nukleinsäureoligomer oder -polymer oder ein Nukleinsäureanalogon und besonders bevorzugt eine peptidische Nukleinsäure verwendet.

Das erfindungsgemäße Verfahren kann in einer solchen Weise durchgeführt werden, daß sämtliche Komponenten, auch die Festphase, zuerst in das Reaktionsgefäß eingebracht werden. Dabei ist es bevorzugt, als Festphasen entsprechend modifizierte Glas- oder Plastikbeads zu verwenden. Der Immunoassay kann jedoch auch so durchgeführt werden, daß als eine der beiden Festphasen das Reaktionsgefäß verwendet wird und als andere der beiden Festphasen Glas- oder Plastikbeads verwendet werden. Auch Festphasen in Form eines Teststreifens können verwendet werden, wobei entweder beide Festphasen als Teststreifen ausgestaltet sind oder das Reaktionsgefäß wiederum eine der beiden Festphasen darstellt.

Die Kopplung von Streptavidin oder Avidin als ein Partner eines spezifischen Bindesystems an die Festphase erfolgt in einer besonders bevorzugten Ausführungsform der Erfindung mittels Thermorinderserumalbumin (TRSA).

In einer weiteren bevorzugten Ausführungsform der Erfindung verwendet man als Festphasen jeweils Mikrotiterplatten. Platte steht hier stellvertretend auch für andere Ausformungen, z.B. 8- und 16-er Streifen. Dabei können mehrere verschiedene Immuno-oder Nukleinsäure-Hybridisierungsassays parallel durchgeführt werden. Zuerst wird dabei die Testprobe gegebenenfalls in Anwesenheit von Nachweis- oder/und Fangreagenz mit einer als inaktivierten Festphase ausgestalteten Mikrotiterplatte in Kontakt gebracht, wobei die Proben in die Vertiefungen eingebracht werden. Nach einer Vorinkubationszeit, in der unspezifische Bindungen mit der inaktivierten Festphase erfolgen können, werden dann mittels Pipette, bevorzugt Mehrkanalpipette die verschiedenen Parallelproben ohne Zeitverzug in eine weitere Mikrotiterplatte übergeführt, welche als aktive Festphase ausgestaltet ist.

Die Pipettierung mit der Mehrkanalpipette hat den Vorteil, daß die Einpipettierung in die Vertiefungen mit aktiver Festphase zeitgleich erfolgt und damit eine erhöhte Testpräzision zu erreichen ist, gegebenenfalls bei gleichzeitig insgesamt kürzerer Testdauer.

Ein weiterer Gegenstand der vorliegenden Erfindung ist eine inaktive Festphase zur Verwendung in Immuno- oder Nukleinsäure-Hybridisierungsassays, die dadurch gekennzeichnet ist, daß sie einer in einem Immuno- oder Nukleinsäure-Hybridisierungsassay zu verwendenden Festphase entspricht, wobei sie aber derart modifiziert ist, daß keine Bindung an ein Fangreagenz stattfinden kann. Unter Fangreagenz ist hierbei wiederum ein mit einer in einem Immuno- oder Nukleinsäure-Hybridisierungsassay nachzuweisenden Substanz spezifisch bindefähiges Reagenz gemeint, welches durch seine Fähigkeit mit einer Festphase zu binden, eine Wandbindung vermitteln kann.

Vorzugsweise ist die inaktivierte Festphase ein Plastikröhrchen, eine Mikrotiterplatte, Glas- oder Plastikbeads oder Latexpartikel.

Die inaktivierte Festphase weist dabei vorzugsweise als Partner eines der Bindepaare Biotin/Streptavidin, Biotin/Avidin, Antigen/Antikörper, Hapten/Antihaptenantikörper oder jeweils miteinander bindefähige Fragmente auf. Diese Partner eines Bindepaares sind dabei nach an sich bekannten Methoden mit der inaktivierten Festphase verbunden, wie beispielsweise durch adsorptive Beschichtung. Es ist hierbei besonders bevorzugt, daß an die Festphase als Partner des Bindepaares Avidin oder Streptavidin gebunden ist, wobei die Kopplung besonders bevorzugt durch Beschichtung mittels Thermo-RSA (TRSA) bewirkt ist. Die Inaktivierung der erfindungsgemäßen inaktiven Festphase erfolgt bevorzugt durch Absättigung von Avidin oder Streptavidin mit Biotin oder einem Biotinderivat, durch kovalente Modifizierung des aktiven Zentrums von Avidin oder Streptavidin, wobei besonders bevorzugte kovalente Modifizierung über eine Derivatisierung mindestens einer Aminosäure des aktiven Zentrums oder eine kovalente Kopplung von Biotin an das aktive Zentrum erfolgt. Als weitere Möglichkeit der Inaktivierung der erfindungsgemäßen inaktiven Festphase steht die kovalente Kopplung von Biotin an das aktive Zentrum durch photoaktivierbares Biotin, beispielsweise Biotin-DADOO-AB und nachfolgende Photoaktivierung zur Verfügung. Auch Verwendung von derartigen Fragmenten von Avidin oder Streptavidin, an die keine spezifische Bindung von Biotin mehr erfolgen kann, an die Festphase und Herstellung einer inaktivierten Festphase auf diese Weise ist im Rahmen der Erfindung möglich. Schließlich ist es im Rahmen der Erfindung besonders bevorzugt, die Inaktivierung des aktiven Zentrums von Avidin oder Streptavidin durch gentechnologische Veränderung, wie Substitution, Deletion oder Insertion, einzelner oder mehrerer Aminosäurereste zu bewirken.

Ein weiterer Gegenstand der vorliegenden Erfindung ist die Verwendung einer erfindungsgemäßen inaktiven Festphase zur Entstörung von Immuno- oder Nukleinsäure-Hybridisierungsassays durch Abfangen von unspezifisch bindenden Komponenten in einer zu analysierenden Probe. Das erfindungsgemäße Verfahren bzw. die erfindungsgemäße Verwendung der inaktivierten Festphase in einem Immuno- oder Nukleinsäure-Hybridisierungsassay erlaubt auf einfache und kostengünstige Weise Störungen durch unspezifisch bindende Komponenten abzufangen und danach einen ungestörten Immunoassay durchzuführen. Falsch positive Resultate werden hierbei ebenso vermieden wie eine zu breite Streuung der Testresultate, was ebenfalls die Sensitivität und Spezifität des betreffenden Testes beeinträchtigt.

Gegenstand der vorliegenden Erfindung ist weiterhin ein Testkit für Immuno- oder Nukleinsäure-Hybridisierungsassays zum qualitativen oder/und quantitativen Nachweis einer Substanz in einer Testprobe, wobei der Testkit die für einen Immuno- oder Nukleinsäure-Hybridisierungsassay üblichen Komponenten und eine inaktivierte Festphase enthält, die weitgehend einer aktiven, im Immuno- oder Nukleinsäure-Hybridisierungsassay verwendeten Festphase entspricht, an die aber das Fangreagenz nicht binden kann. Bevorzugt enthält der Testkit folgende Komponenten:
a) ein Fangreagenz, das mittels zweier verschiedener Bindungsstellen 1) gegebenenfalls zusammen mit weiteren Testkomponenten einen spezifischen Nachweis der zu bestimmenden Substanz ermöglicht und 2) über ein spezifisches Bindungspaar, dessen einer Partner mit dem Fangreagenz verbunden ist und dessen zweiter Partner an eine aktive Festphase gekoppelt ist, an eine aktive Festphase gebunden oder bindefähig ist,
b) ein Nachweisreagenz,
c) eine aktive Festphase und
d) eine erfindungsgemäße inaktivierte Festphase.

Ein weiterer Gegenstand der vorliegenden Erfindung ist ein Verfahren zur Vermeidung der Störung durch unspezifische Bindungen an die Festphase in einem Immunoassay unter Einsatz der folgenden Komponenten
a) ein Fangreagenz, das mittels zweier verschiedener Bindungsstellen 1) gegebenenfalls zusammen mit weiteren Testkomponenten einen spezifischen Nachweis der zu bestimmenden Substanz ermöglicht und 2) über ein spezifisches Bindungspaar, dessen einer Partner mit dem Fangreagenz verbunden ist und dessen zweiter Partner an eine aktive Festphase gekoppelt ist, an eine aktive Festphase gebunden oder bindefähig ist,
b) mindestens zwei aktive Festphasen, dadurch gekennzeichnet, daß vor Zugabe von Fangreagenz und gegebenenfalls weiteren Testkomponenten eine Inkubation der Testprobe in Gegenwart einer ersten aktiven Festphase erfolgt, und danach die Testprobe von dieser ersten Festphase abgetrennt wird und dann eine Inkubation in Gegenwart von Fangreagenz und gegebenenfalls weiteren Testkomponenten und einer zweiten, weitgehend identischen Festphase erfolgt und die zu bestimmende Substanz in geeigneter Weise nachgewiesen wird. Auch dieses Verfahren ermöglicht es, unspezifische Störungen durch Bindung von in der Testprobe enthaltenen Substanzen an die Festphase und damit die Erzeugung eines unspezifischen Signals zu vermeiden. Durch die Vorinkubation mit einer aktiven Festphase, die der im Immunoassay verwendeten Festphase entspricht, werden unspezifisch bindende Substanzen vorab weggefangen, wonach erst nach einer Abtrennung der Festphase und nachfolgender Inkubation mit den Komponenten Fangreagenz, gegebenenfalls Nachweisreagenz und neuer, unbelegter Testphase der eigentliche Immunoassay durchgeführt wird. Eine Anbindung der zu bestimmenden Substanz an die aktive Festphase kann während der Vorinkubation noch nicht erfolgen, da das Fangreagenz erst in der nachfolgenden zweiten Inkubation zugegeben wird. Eine Inaktivierung der Festphase ist bei dieser Ausführungsform nicht unbedingt notwendig.

Das folgende Beispiel soll die Erfindung weiter erläutern:

### Beispiel 1

Vergleichende Testführung eines Immunoassays zur Bestimmung von Anti-GAD-Antikörpern in Humanserum:
A) Testführung nach bisher angewandten Methoden: 100 µl einer Lösung bestehend aus biotinylierter Glutamatdecarboxylase (GAD) in einer Konzentration von ca. 1 µg/ml werden jeweils in eine Vertiefung einer aktiven Streptavidin-beschichteten Mikrotiterplatte pipettiert und eine Stunde bei Raumtemperatur inkubiert. Danach wird die Platte dreimal mit einem geeigneten Puffer gewaschen. Humanserum wird 1+25 verdünnt in Inkubationspuffer aus dem Enzymun-Test ^{R} Anti-HIV 1+2, wovon wiederum 100 µl in die jeweiligen Vertiefungen der Mikrotiterplatte einpipettiert werden und eine Stunde bei Raumtemperatur inkubiert wird. Auch nach diesem Schritt werden die Proben abgesaugt und die Vertiefungen dreimal gewaschen. Der POD-gekoppelte Nachweisantikörper aus Schaf mit Bindungsspezifität für Human-IgG (Schaf-(Anti-)Human-POD) wird in Konjugatpuffer aus dem Enzymun-Test^{R} Anti-HIV 1+2 auf eine Konzentration von ca. 75 mU/ml verdünnt und in jede Vertiefung der Mikrotiterplatte 100 µl dieser verdünnten Lösung zugegeben und eine Stunde bei Raumtemperatur inkubiert. Die Flüssigkeit wird wiederum abgesaugt, die Platte dreimal gewaschen wie oben. Der Farbstoff ABTS^{R} wird zu einer Konzentration von 1 mg/ml im Enzymun-Test^{R} Substratpuffer gelöst und 100 µl pro Vertiefung der Mikroplatte einpipettiert und inkubiert. Nach ca. 30 min. wird die Extinktion in einem Mikrotiter-Plattenphotometer abgelesen. Meßwellenlänge ist 405 nm und die Referenzwellenlänge 492 nm. Der Leerwert wird ermittelt aus zwei unbehandelte Küvetten, die nur Substrat-/Farbstofflösung enthalten. Die Ergebnisse für die GAD-Bestimmung mit acht verschiedenen Seren ist in Tabelle 1 gezeigt in der Spalte "ohne Vorinkubation".
B) Testführung nach dem erfindungsgemäßen Verfahren
   Eine Lösung aus biotinylierter GAD (ca. 1 µg/ml) und Serum (1:26) wird in geeignetem Puffer, wie unter 1A) beschrieben hergestellt, und in einer mit inaktiviertem Streptavidin beschichteten Mikrotiterplatte in einer Menge von 250 µl dieser Lösung pro Vertiefung eine Stunde inkubiert. Aus einer Vertiefung werden zweimal jeweils 100 µl in eine Vertiefung einer aktiven Streptavidin-beschichteten Platte pipettiert und wiederum eine Stunde inkubiert. Danach wird die Lösung abpipettiert, dreimal mit Puffer gewaschen und die Inkubation mit Schaf-(Anti-)Human-POD sowie die Farbbildungsreaktion gemäß 1A) durchgeführt. Die gemäß dem erfindungsgemäßen Vorgehen erhaltenen Werte sind aus Tabelle 1 zu entnehmen, aus der Spalte die mit "mit Inkubation" bezeichnet ist.

Die dramatische Reduktion der unspezifischen Bindung beim erfindungsgemäßen Vorgehen geht aus diesen Daten deutlich hervor.

**Tabelle 1**

| Störseren | mit Inkubation | ohne Vorinkubation |
|---|---|---|
| 7478-262 | 15 | 260 |
| -269 | 45 | 468 |
| -272 | 30 | 1669 |
| -289 | 69 | 2208 |
| 7480-203 | 97 | 1343 |
| -210 | 72 | 136 |
| 7463-531 | 0 | 1613 |
| 7478-298 | 182 | 1517 |

## Patentansprüche

1. Verfahren zum qualitativen oder/und quantitativen Nachweis einer zu bestimmenden Substanz in einer Testprobe mit Hilfe eines Immuno-oder Nukleinsäure-Hybridisierungsassays, bei dem die folgenden Komponenten eingesetzt werden:
a) ein Fangreagenz, das mittels zweier verschiedener Bindungsstellen 1) gegebenenfalls zusammen mit weiteren Testkomponenten einen spezifischen Nachweis der zu bestimmenden Substanz ermöglicht und 2) über ein spezifisches Bindungspaar, dessen einer Partner mit dem Fangreagenz verbunden ist und dessen zweiter Partner an eine aktive Festphase gekoppelt ist, an eine aktive Festphase gebunden oder bindefähig ist,
b) eine aktive Festphase, an die der zweite Partner des spezifischen Bindungspaares gekoppelt ist, und
c) eine inaktive Festphase, die der aktiven Festphase entspricht, die jedoch so modifiziert ist, dass das Fangreagenz nicht daran binden kann,
wobei die Testprobe entweder zuerst mit der inaktiven Festphase alleine und erst später mit der aktiven Festphase oder gleichzeitig mit aktiver und inaktiver Festphase in Kontakt gebracht wird, wobei das Fangreagenz und gegebenenfalls weitere Testkomponenten entweder von Anfang an anwesend sind oder aber erst später zugegeben werden, und ein Nachweis der zu bestimmenden Substanz erfolgt.

2. Verfahren nach Anspruch 1,
**dadurch gekennzeichnet,**
**dass** ein Nachweisreagenz eingesetzt wird, das zusammen mit dem Fangreagenz einen spezifischen Nachweis der zu bestimmenden Substanz ermöglicht und eine indirekte oder direkte Markierung trägt und der Nachweis der zu bestimmenden Substanz durch Bestimmung der Markierung an der aktiven Festphase oder/und im Testprobenüberstand erfolgt.

3. Verfahren nach Anspruch 1 oder 2,
**dadurch gekennzeichnet,**
**dass** als Festphase Plastikröhrchen, Mikrotiterplatten, Glas- oder Plastikbeads oder Latexpartikel verwendet werden.

4. Verfahren nach Anspruch 3,
**dadurch gekennzeichnet,**
**dass** man zumindest als inaktive Festphase Glas- oder Plastikbeads verwendet, die gegebenenfalls entsprechend modifiziert sind .

5. Verfahren nach einem der Ansprüche 1 bis 4,
**dadurch gekennzeichnet,**
**dass** die Testprobe zuerst mit Fangreagenz und der inaktiven Festphase inkubiert wird, die inaktive Festphase dann entfernt wird und erst danach die Inkubation mit der aktiven Festphase und dem Nachweisreagenz erfolgt.

6. Verfahren nach einem der Ansprüche 1 bis 5,
**dadurch gekennzeichnet,**
**dass** man als spezifisches Bindepaar Biotin/Avidin-, Biotin/Streptavidin-, Antigen/Antikörper-, Hapten/Anti-Hapten-Antikörper-Paare bzw. miteinander spezifisch bindefähige Fragmente hiervon verwendet.

7. Verfahren nach einem der vorhergehenden Ansprüche,
**dadurch gekennzeichnet,**
**dass** man als spezifisches Bindepaar Biotin/Streptavidin oder Biotin/Avidin verwendet, wobei Avidin bzw. Streptavidin an die Festphase gebunden vorliegen.

8. Verfahren nach Anspruch 7,
**dadurch gekennzeichnet,**
**dass** bei der Herstellung der inaktiven Festphase eine Inaktivierung von Avidin oder Streptavidin durch Absättigung mit Biotin oder einem Biotinderivat bewirkt wird.

9. Verfahren nach Anspruch 7,
**dadurch gekennzeichnet,**
**dass** die Inaktivierung von Avidin oder Streptavidin durch kovalente Modifizierung des aktiven Zentrums erfolgt.

10. Verfahren nach Anspruch 9,
**dadurch gekennzeichnet,**
**dass** zur kovalenten Modifizierung eine Derivatisierung mindestens einer Aminosäure des aktiven Zentrums oder eine kovalente Kopplung von Biotin an das aktive Zentrum durchgeführt wird.

11. Verfahren nach Anspruch 9 oder 10,
**dadurch gekennzeichnet,**
**dass** die kovalente Kopplung von Biotin an das aktive Zentrum durch photoaktivierbares Biotin, beispielsweise Biotin-DADOO-AB erfolgt.

12. Verfahren nach Anspruch 7,
**dadurch gekennzeichnet,**
**dass** bei der Herstellung der inaktiven Festphase eine Inaktivierung von Avidin oder Streptavidin durch gentechnologische Veränderung des aktiven Zentrums, wie Substitution, Deletion oder Insertion einzelner oder mehrerer Aminosäurereste, erfolgt.

13. Verfahren nach einem der vorhergehenden Ansprüche,
**dadurch gekennzeichnet,**
**dass** man als Testprobe eine Körperflüssigkeit und insbesondere Blut, Blutplasma, Serum, Speichel, Gewebsflüssigkeit, Liquor oder Urin verwendet.

14. Verfahren nach einem der vorhergehenden Ansprüche,
**dadurch gekennzeichnet,**
**dass** man zum Nachweis der zu bestimmenden Substanz eine Markierung ausgewählt aus radioaktiven Markierungen, Enzymmarkierungen, Fluoreszenzmarkierungen, Chemilumineszenzmarkierungen, Elektrochemilumineszenzmarkierungen und gefärbten Partikeln verwendet.

15. Verfahren nach einem der vorhergehenden Ansprüche,
**dadurch gekennzeichnet,**
**dass** man für den Fall, dass es sich bei der zu bestimmenden Substanz um ein Antigen handelt, als Fangreagenz Antikörper oder Antikörperfragmente verwendet.

16. Verfahren nach einem der Ansprüche 1 bis 14,
**dadurch gekennzeichnet,**
**dass** man für den Fall, dass es sich bei der zu bestimmenden Substanz um einen Antikörper handelt, als Fangreagenz Antigene oder/und Haptene verwendet.

17. Verfahren nach einem der Ansprüche 1 bis 14,
**dadurch gekennzeichnet,**
**dass** man für den Fall, dass es sich bei der zu bestimmenden Substanz um eine Nukleinsäure handelt, als Fangreagenz ein Nukleinsäureoligomer oder -polymer oder ein Nukleinsäureanalogon verwendet.

18. Verfahren nach Anspruch 16,
**dadurch gekennzeichnet,**
**dass** man als Nukleinsäureanalogon eine peptidische Nukleinsäure verwendet.

19. Verfahren nach einem der vorhergehenden Ansprüche,
**dadurch gekennzeichnet,**
**dass** man ein Nachweisreagenz verwendet, das spezifisch an die zu bestimmende Substanz bindet.

20. Verfahren nach Anspruch 19,
**dadurch gekennzeichnet,**
**dass** man für den Fall, dass es sich bei der zu bestimmenden Substanz um ein Antigen handelt, als Nachweisreagenz Antikörper oder Antikörperfragmente verwendet.

21. Verfahren nach Anspruch 19,
**dadurch gekennzeichnet,**
**dass** man für den Fall, dass es sich bei der zu bestimmenden Substanz um einen Antikörper handelt, als Nachweisreagenz Antigene oder/und Haptene verwendet.

22. Verfahren nach Anspruch 19,
**dadurch gekennzeichnet,**
**dass** man für den Fall, dass es sich bei der zu bestimmenden Substanz um eine Nukleinsäure handelt, als Nachweisreagenz ein Nukleinsäureoligomer oder -polymer oder ein Nukleinsäureanalogon verwendet.

23. Verfahren nach Anspruch 22,
**dadurch gekennzeichnet,**
**dass** man als Nukleinsäureanalogon eine peptidische Nukleinsäure verwendet.

24. Verfahren nach einem der Ansprüche 1 bis 18,
**dadurch gekennzeichnet,**
**dass** man ein Nachweisreagenz verwendet, das mit der zu bestimmenden Substanz um eine Bindungsstelle konkurriert.

25. Verfahren nach Anspruch 24,
**dadurch gekennzeichnet,**
**dass** man für den Fall, dass es sich bei der zu bestimmenden Substanz um einen Antikörper handelt, als Nachweisreagenz Antikörper oder Antikörperfragmente verwendet.

26. Verfahren nach Anspruch 24,
**dadurch gekennzeichnet,**
**dass** man für den Fall, dass es sich bei der zu bestimmenden Substanz um ein Antigen handelt, als Nachweisreagenz Antigene oder/und Haptene verwendet.

27. Verfahren nach Anspruch 24,
**dadurch gekennzeichnet,**
**dass** man für den Fall, dass es sich bei der zu bestimmenden Substanz um eine Nukleinsäure handelt, als Nachweisreagenz ein Nukleinsäureoligomer oder -polymer oder ein Nukleinsäureanalogon verwendet.

28. Verfahren nach Anspruch 27,
**dadurch gekennzeichnet,**
**dass** man als Nukleinsäureanalogon eine peptidische Nukleinsäure verwendet.

29. Verfahren nach einem der vorhergehenden Ansprüche,
**dadurch gekennzeichnet,**
**dass** die Kopplung von Streptavidin oder Avidin an die Festphase mittels Thermorinderserumalbumin (TRSA) erfolgt.

30. Verfahren nach einem der vorhergehenden Ansprüche,
**dadurch gekennzeichnet,**
**dass** man als inaktive Festphase eine Mikrotiterplatte verwendet, deren Vertiefungen mit verschiedenen Testproben gefüllt werden, und bevorzugt mittels Mehrkanalpipette diese verschiedenen Testproben ohne Zeitverzug in eine weitere Mikrotiterplatte als aktive Festphase überführt.

31. Verfahren nach Anspruch 30,
**dadurch gekennzeichnet,**
**dass** bei der Vorinkubation mit inaktiver Festphase bereits auch Fangreagenz und gegebenenfalls bereits auch Nachweisreagenz anwesend ist.

32. Inaktive Festphase zur Verwendung in Immuno- oder Nukleinsäure-Hybridisierungsassays zum qualitativen oder/und quantitativen Nachweis einer zu bestimmenden Substanz in einer Testprobe, bei dem die folgenden Komponenten eingesetzt werden:
a) ein Fangreagenz, das mittels zweier verschiedener Bindungsstellen 1) gegebenenfalls zusammen mit weiteren Testkomponenten einen spezifischen Nachweis der zu bestimmenden Substanz ermöglicht und 2) über ein spezifisches Bindungspaar, dessen einer Partner mit dem Fangreagenz verbunden ist und dessen zweiter Partner an eine aktive Festphase gekoppelt ist, an eine aktive Festphase gebunden oder bindefähig ist,
b) eine aktive Festphase, an die der zweite Partner des spezifischen Bindungspaares gekoppelt ist,
**dadurch gekennzeichnet,**
**dass** die inaktive Festphase der aktiven Festphase entspricht, sie jedoch derart modifiziert ist, dass keine Bindung an das Fangreagenz stattfinden kann.

33. Inaktive Festphase nach Anspruch 32,
**dadurch gekennzeichnet,**
**dass** sie als Festphase ein Plastikröhrchen, eine Mikrotiterplatte, Glasoder Plastikbeads oder Latexpartikel aufweist.

34. Inaktive Festphase nach Anspruch 32 oder 33,
**dadurch gekennzeichnet,**
**dass** sie einen Partner eines der Bindepaare Biotin/Streptavidin, Biotin/Avidin, Antigen/Antikörper, Hapten/Anti-Hapten-Antikörper oder jeweils miteinander bindefähiger Fragmente umfasst.

35. Inaktive Festphase nach Anspruch 34,
**dadurch gekennzeichnet,**
**dass** der Partner des Bindepaares Avidin oder Streptavidin ist.

36. Inaktive Festphase nach Anspruch 35,
**dadurch gekennzeichnet,**
**dass** die Kopplung von Avidin oder Streptavidin an die Festphase durch Beschichtung mittels Thermo-RSA bewirkt ist.

37. Inaktive Festphase nach Anspruch 35 oder 36,
**dadurch gekennzeichnet,**
**dass** die Inaktivierung durch Absättigung von Avidin oder Streptavidin mit Biotin oder einem Biotinderivat erfolgt ist.

38. Inaktive Festphase nach Anspruch 35 oder 36,
**dadurch gekennzeichnet,**
**dass** die Inaktivierung durch kovalente Modifizierung des aktiven Zentrums von Avidin oder Streptavidin erfolgt ist.

39. Inaktive Festphase nach Anspruch 38,
**dadurch gekennzeichnet,**
**dass** zur kovalenten Modifizierung eine Derivatisierung mindestens einer Aminosäure des aktiven Zentrums oder eine kovalente Kopplung von Biotin an das aktive Zentrum erfolgt ist.

40. Inaktive Festphase nach Anspruch 39,
**dadurch gekennzeichnet,**
**dass** die kovalente Kopplung von Biotin an das aktive Zentrum durch photoaktivierbares Biotin, beispielsweise Biotin-DADOO-AB erfolgt ist.

41. Inaktive Festphase nach Anspruch 35 oder 36,
**dadurch gekennzeichnet,**
**dass** die Inaktivierung durch gentechnologische Veränderung des aktiven Zentrums von Avidin oder Streptavidin, wie Substitution, Deletion oder Insertion einzelner oder mehrerer Aminosäurereste, erfolgt ist.

42. Verwendung einer inaktiven Festphase nach einem der Ansprüche 32 bis 41 zur Entstörung von Immuno- oder Nukleinsäure-Hybridisierungsassays durch Abfangen von unspezifisch bindenden Komponenten in einer zu analysierenden Testprobe.

43. Testkit für einen Immuno- oder Nukleinsäure-Hybridisierungsassay zum qualitativen oder/und quantitativen Nachweis einer zu bestimmenden Substanz in einer Testprobe, enthaltend die Komponenten des jeweiligen Immuno- oder Nukleinsäure-Hybridisierungsassays sowie eine inaktivierte Festphase gemäß einem der Ansprüche 32 bis 41.

44. Testkit nach Anspruch 43,
**dadurch gekennzeichnet,**
**dass** er folgende Komponenten umfasst:
a) ein Fangreagenz, das mittels zweier verschiedener Bindungsstellen 1) gegebenenfalls zusammen mit weiteren Testkomponenten einen spezifischen Nachweis der zu bestimmenden Substanz ermöglicht und 2) über ein spezifisches Bindungspaar, dessen einer Partner mit dem Fangreagenz verbunden ist und dessen zweiter Partner an eine aktive Festphase gekoppelt ist, an eine aktive Festphase gebunden oder bindefähig ist,
b) ein Nachweisreagenz,
c) eine aktive Phase, an die der zweite Partner des spezifischen Bindungspaares gekoppelt ist, und
d) eine inaktive Festphase, die der aktiven Festphase entspricht, die jedoch so modifiziert ist, dass das Fangreagenz nicht binden kann.

45. Verfahren zur Vermeidung der Störung durch unspezifische Bindungen an die Festphase in einem Immuno- oder Nukleinsäure-Hybridisierungsassay unter Einsatz der folgenden Komponenten
a) ein Fangreagenz, das mittels zweier verschiedener Bindungsstellen 1) gegebenenfalls zusammen mit weiteren Testkomponenten einen spezifischen Nachweis der zu bestimmenden Substanz ermöglicht und 2) über ein spezifisches Bindungspaar, dessen einer Partner mit dem Fangreagenz verbunden ist und dessen zweiter Partner an eine aktive Festphase gekoppelt ist, an eine aktive Festphase gebunden oder bindefähig ist,
b) mindestens zwei aktive Festphasen,
**dadurch gekennzeichnet,**
**dass** vor Zugabe von Fangreagenz und gegebenenfalls weiteren Testkomponenten eine Inkubation der Testprobe in Gegenwart einer ersten aktiven Festphase erfolgt, und danach die Testprobe von dieser ersten Festphase abgetrennt wird und dann eine Inkubation in Gegenwart von Fangreagenz und gegebenenfalls weiteren Testkomponenten und einer zweiten, weitgehend identischen Festphase erfolgt und die zu bestimmende Substanz in geeigneter Weise nachgewiesen wird.

## Claims

1. Method for the qualitative or/and quantitative detection of a substance to be determined in a test sample with the aid of an immunoassay or nucleic acid hybridization assay in which the following components are used:
a) a capture reagent which enables a specific detection of the substance to be determined by means of two different binding sites 1) optionally together with further test components and 2) is bound or is capable of binding to an active solid phase via a specific binding pair one partner of which is linked to the capture reagent and the second partner of which is coupled to an active solid phase
b) an active solid phase to which the second partner of the specific binding pair is coupled and
c) an inactive solid phase which corresponds to the active solid phase but is modified in such a manner that the capture reagent cannot bind to it,
wherein the test sample is either firstly brought into contact with the inactive solid phase alone and only later with the active solid phase or is simultaneously brought into contact with the active and inactive solid phase during which the capture reagent and optionally further test components are either present from the start or are only added later and the substance to be determined is detected.

2. Method as claimed in claim 1,
wherein
a detection reagent is used which together with the capture reagent enables a specific detection of the substance to be determined and carries an indirect or direct label and the substance to be determined is detected by determination of the label on the active solid phase or/and in the test sample supernatant.

3. Method as claimed in claim 1 or 2,
wherein
plastic tubes, microtitre plates, glass or plastic beads or latex particles are used as the solid phase.

4. Method as claimed in claim 3,
wherein
glass or plastic beads that are optionally modified accordingly are at least used as the inactive solid phase.

5. Method as claimed in one of the claims 1 to 4;
wherein
the test sample is firstly incubated with the capture reagent and the inactive solid phase, the inactive solid phase is then removed and only afterwards incubated with the active solid phase and the detection reagent.

6. Method as claimed in one of the claims 1 to 5,
wherein
biotinlavidin, biotin/streptavidin, antigen/ antibody, hapten/anti-hapten antibody pairs or fragments thereof that are capable of specific binding to each other are used as the specific binding pair.

7. Method as claimed in one of the previous claims,
wherein
biotin/streptavidin or biotin/avidin is used as the specific binding pair in which avidin or streptavidin is present bound to the solid phase.

8. Method as claimed in claim 7,
wherein
during the production of the inactive solid phase avidin or streptavidin are inactivated by saturation with biotin or a biotin derivative.

9. Method as claimed in claim 7,
wherein
avidin or streptavidin are inactivated by covalent modification of the active centre.

10. Method as claimed in claim 9,
wherein
at least one amino acid of the active centre is derivatized or biotin is covalently coupled to the active centre for the covalent modification.

11. Method as claimed in claim 9 or 10,
wherein
the covalent coupling of biotin to the active centre is carried out using biotin that can be photoactivated for example biotin-DADOO-AB.

12. Method as claimed in claim 7,
wherein
during the production of the inactive solid phase avidin or streptavidin is inactivated by modifying the active centre by means of genetic engineering such as by substituting, deleting or inserting individual or several amino acid residues.

13. Method as claimed in one of the previous claims,
wherein
a body fluid and in particular blood, blood plasma, serum, saliva, tissue fluid, liquor or urine is used as the test sample.

14. Method as claimed in one of the previous claims,
wherein
in order to detect the substance to be determined a label is used which is selected from radioactive labels, enzyme labels, fluorescent labels, chemiluminescent labels, electrochemiluminescent labels and dyed particles.

15. Method as claimed in one of the previous claims,
wherein
antibodies or antibody fragments are used as the capture reagent in the case that the substance to be determined is an antigen.

16. Method as claimed in one of the claims 1 to 14,
wherein
antigens or/and haptens are used as the capture reagent in the case that the substance to be determined is an antibody.

17. Method as claimed in one of the claims 1 to 14,
wherein
a nucleic acid oligomer or nucleic acid polymer or a nucleic acid analogue is used as the capture reagent in the case that the substance to be determined is a nucleic acid.

18. Method as claimed in claim 16,
wherein
a peptidic nucleic acid is used as the nucleic acid analogue.

19. Method as claimed in one of the previous claims,
wherein
a detection reagent is used that specifically binds to the substance to be determined.

20. Method as claimed in claim 19,
wherein
antibodies or antibody fragments are used as the detection reagent in the case that the substance to be determined is an antigen.

21. Method as claimed in claim 19,
wherein
antigens or/and haptens are used as the detection reagent in the case that the substance to be determined is an antibody.

22. Method as claimed in claim 19,
wherein
a nucleic acid oligomer or nucleic acid polymer or a nucleic acid analogue is used as the detection reagent in the case that the substance to be determined is a nucleic acid.

23. Method as claimed in claim 22,
wherein
a peptidic nucleic acid is used as the nucleic acid analogue.

24. Method as claimed in one of the claims 1 to 18,
wherein
a detection reagent is used which competes with the substance to be determined for a binding site.

25. Method as claimed in claim 24,
wherein
antibodies or antibody fragments are used as the detection reagent in the case that the substance to be determined is an antibody.

26. Method as claimed in claim 24,
wherein
antigens or/and haptens are used as the detection reagent in the case that the substance to be determined is an antigen.

27. Method as claimed in claim 24,
wherein
a nucleic acid oligomer or nucleic acid polymer or a nucleic acid analogue is used as the detection reagent in the case that the substance to be determined is a nucleic acid.

28. Method as claimed in claim 27,
wherein
a peptidic nucleic acid is used as the nucleic acid analogue.

29. Method as claimed in one of the previous claims,
wherein
streptavidin or avidin are coupled to the solid phase by means of thermo-bovine-serum-albumin (TBSA).

30. Method as claimed in one of the previous claims,
wherein
a microtitre plate is used as the inactive solid phase the wells of which are filled with various test samples and these various test samples are transferred to a further microtitre plate as the active solid phase without time delay preferably by means of a multichannel pipette.

31. Method as claimed in claim 30,
wherein
the capture reagent and optionally the detection reagent are also already present during the preincubation with inactive solid phase.

32. Inactive solid phase for use in immunoassays or nucleic acid hybridization assays for the qualitative or/and quantitative detection of a substance to be determined in a test sample in which the following components are used:
a) a capture reagent which enables a specific detection of the substance to be determined by means of two different binding sites 1) optionally together with further test components and 2) is bound or is capable of binding to an active solid phase via a specific binding pair one partner of which is linked to the capture reagent and the second partner of which is coupled to an active solid phase
b) an active solid phase to which the second partner of the specific binding pair is coupled
wherein
the inactive solid phase corresponds to the active solid phase but is modified in such a manner that the capture reagent cannot bind to it.

33. Inactive solid phase as claimed in claim 32,
wherein
it has a plastic tube, a microtitre plate, glass or plastic beads or latex particles as the solid phase.

34. Inactive solid phase as claimed in claim 32 or 33,
wherein
it comprises a partner of one of the binding pairs biotin/streptavidin, biotin/avidin, antigen/antibody, hapten/anti-hapten antibody or fragments that are capable of binding to each other.

35. Inactive solid phase as claimed in claim 34,
wherein
the partner of the binding pair is avidin or streptavidin.

36. Inactive solid phase as claimed in claim 35,
wherein
avidin or streptavidin are coupled to the solid phase by coating with thermo-BSA.

37. Inactive solid phase as claimed in claim 35 or 36,
wherein
the inactivation is achieved by saturating avidin or streptavidin with biotin or a biotin derivative.

38. Inactive solid phase as claimed in claim 35 or 36,
wherein
the inactivation is achieved by covalent modification of the active centre of avidin or streptavidin.

39. Inactive solid phase as claimed in claim 38,
wherein
at least one amino acid of the active centre is derivatized or biotin is covalently coupled to the active centre for the covalent modification.

40. Inactive solid phase as claimed in claim 39,
wherein
the covalent coupling of biotin to the active centre is achieved by using biotin that can be photoactivated for example biotin-DADOO-AB.

41. Inactive solid phase as claimed in claim 35 or 36,
wherein
the inactivation is achieved by modifying the active centre of avidin or streptavidin by means of genetic engineering such as by substituting, deleting or inserting individual or several amino acid residues.

42. Use of an inactive solid phase as claimed in one of the claims 32 to 41 for eliminating interference of immunoassays or nucleic acid hybridization assays by capturing components that bind unspecifically in a test sample to be analysed.

43. Test kit for an immunoassay or nucleic acid hybridization assay for the qualitative or/and quantitative detection of a substance to be determined in a test sample containing the components of the respective immunoassay or nucleic acid hybridization assay as well as an inactive solid phase as claimed in one of the claims 32 to 41.

44. Test kit as claimed in claim 43,
wherein
it comprises the following components
a) a capture reagent which enables a specific detection of the substance to be determined by means of two different binding sites 1) optionally together with further test components and 2) is bound or is capable of binding to an active solid phase via a specific binding pair one partner of which is linked to the capture reagent and the second partner of which is coupled to an active solid phase
b) a detection reagent
c) an active solid phase to which the second partner of the specific binding pair is coupled and
d) an inactive solid phase which corresponds to the active solid phase but is modified in such a manner that the capture reagent cannot bind to it.

45. Method for avoiding interference by unspecific binding to the solid phase in an immunoassay or nucleic acid hybridization assay using the following components
a) a capture reagent which enables a specific detection of the substance to be determined by means of two different binding sites 1) optionally together with further test components and 2) is bound or is capable of binding to an active solid phase via a specific binding pair one partner of which is linked to the capture reagent and the second partner of which is coupled to an active solid phase
b) at least two active solid phases,
wherein
before addition of the capture reagent and if desired of further test components the test sample is incubated in the presence of a first active solid phase and afterwards the test sample is separated from this first solid phase and then an incubation is carried out in the presence of a capture reagent and optionally further test components and a second, substantially identical solid phase and the substance to be determined is detected in a suitable manner.

## Revendications

1. Procédé pour détecter qualitativement et/ou quantitativement une substance à déterminer dans un échantillon d'essai à l'aide d'un immunoessai ou d'un essai d'hybridation d'acides nucléiques, pour lequel on utilise les composants suivants :
a) un réactif de captage qui à l'aide de deux sites de liaison différents 1) le cas échéant conjointement avec d'autres composants d'essai permet de détecter spécifiquement la substance à déterminer et 2) est lié ou susceptible d'être lié à une phase solide active au moyen d'un couple ou paire de liaisons spécifique dont l'un des partenaires est lié au réactif de captage et dont l'autre partenaire est couplé à une phase solide active,
b) une phase solide active à laquelle est couplé le deuxième partenaire de la paire de liaisons spécifique et
c) une phase solide inactive qui correspond à la phase solide active mais qui est modifiée de telle sorte que le réactif de captage ne peut pas y être lié,
l'échantillon d'essai étant mis en contact soit d'abord avec la phase solide inactive seule et seulement plus tard avec la phase solide active soit simultanément avec les phases solides active et inactive, le réactif de captage et éventuellement d'autres composants d'essai étant soit présents depuis le début soit ajoutés seulement plus tard une détection de la substance à déterminer étant effectuée.

2. Procédé selon la revendication 1 **caractérisé en ce que** l'on utilise un réactif de détection qui conjointement avec le réactif de captage permet une détection spécifique de la substance à déterminer et qui porte un marquage direct ou indirect et **en ce que** la détection de la substance à déterminer est réalisée par détermination du marquage sur la phase solide active et/ou dans le surnageant d'échantillon d'essai.

3. Procédé selon la revendication 1 ou 2 **caractérisé en ce qu'**en tant que phase solide on utilise de petits tubes en plastique, des plateaux à micro-titration, des perles en verre ou en plastique ou des particules en latex.

4. Procédé selon la revendication 3 **caractérisé en ce qu'**au moins en tant que phase solide inactive on utilise des perles en verre ou en plastique qui le cas échéant sont modifiées en conséquence.

5. Procédé selon l'une des revendications 1 à 4 **caractérisé en ce que** l'échantillon d'essai est d'abord incubé avec le réactif de captage et avec la phase solide inactive, la phase solide inactive étant ensuite éliminée et l'incubation est réalisée seulement après avec la phase solide active et avec le réactif de détection.

6. Procédé selon l'une des revendications 1 à 5 **caractérisé en ce que** l'on utilise en tant que paire de liaisons spécifique les paires biotine / avidine, biotine / streptavidine, antigène / anticorps, haptène / anti-haptène anticorps ou des fragments de ces paires susceptibles de se lier spécifiquement les uns aux autres.

7. Procédé selon l'une des revendications précédentes **caractérisé en ce que** l'on utilise en tant que paire de liaisons spécifique la biotine / streptavidine ou la biotine / avidine, l'avidine ou la streptavidine étant alors présente sous forme liée à la phase solide.

8. Procédé selon la revendication 7 **caractérisé en ce que** lors de la préparation de la phase solide inactive on provoque une inactivation de l'avidine ou de la streptavidine par saturation avec de la biotine ou avec un dérivé de la biotine.

9. Procédé selon la revendication 7 **caractérisé en ce que** l'inactivation de l'avidine ou de la streptavidine est réalisée par une modification covalente du centre actif.

10. Procédé selon la revendication 9 **caractérisé en ce que** pour la modification covalente on réalise une dérivation d'au moins un acide aminé du centre actif ou un couplage covalent de biotine sur le centre actif.

11. Procédé selon la revendication 9 ou 10 **caractérisé en ce que** le couplage covalent de la biotine sur le centre actif est réalisé par de la biotine photoactivable, par exemple de la biotine DADOO-AB.

12. Procédé selon la revendication 7 **caractérisé en ce que** lors de la préparation de la phase solide inactive il se produit une inactivation de l'avidine ou de la streptavidine par modification génétique du centre actif comme la substitution, la suppression ou l'insertion d'un ou de plusieurs restes d'acide aminé.

13. Procédé selon l'une des revendications précédentes **caractérisé en ce que** l'on utilise en tant qu'échantillon d'essai un liquide corporel, en particulier le sang, le plasma sanguin, le sérum, la salive, les liquides tissulaires, la liqueur ou l'urine.

14. Procédé selon l'une des revendications précédentes **caractérisé en ce que** pour détecter la substance à déterminer on chosit un marquage parmi les marquages radioactifs, les marquages enzymatiques, les marquages fluorescents, les marquages chimioluminescents, les marquages électro-chimioluminescents et les particules colorées.

15. Procédé selon l'une des revendications précédentes **caractérisé en ce que** l'on utilise en tant que réactif de captage des anticorps ou des fragments d'anticorps lorsque la substance à déterminer est un antigène.

16. Procédé selon l'une des revendications 1 à 14 **caractérisé en ce que** l'on utilise en tant que réactif de captage des antigènes et/ou des haptènes lorsque la substance à déterminer est un anticorps.

17. Procédé selon l'une des revendications 1 à 14 **caractérisé en ce que** l'on utilise en tant que réactif de captage un oligomère ou un polymère d'acide nucléique ou un analogue d'acide nucléique lorsque la substance à déterminer est un acide nucléique.

18. Procédé selon la revendication 16 **caractérisé en ce que** l'on utilise un acide nucléique peptidique en tant qu'analogue d'acide nucléique.

19. Procédé selon l'une des revendications précédentes **caractérisé en ce que** l'on utilise un réactif de détection qui se lie spécifiquement à la substance à déterminer.

20. Procédé selon la revendication 19 **caractérisé en ce que** l'on utilise en tant que réactif de détection des anticorps ou des fragments d'anticorps lorsque la substance à déterminer est un antigène.

21. Procédé selon la revendication 19 **caractérisé en ce que** l'on utilise en tant que réactif de détection des antigènes et/ou des haptènes lorsque la substance à déterminer est un anticorps.

22. Procédé selon la revendication 19 **caractérisé en ce que** l'on utilise en tant que réactif de détection un oligomère ou un polymère d'acide nucléique ou un analogue d'acide nucléique lorsque la substance à déterminer est un acide nucléique.

23. Procédé selon la revendication 22 **caractérisé en ce que** l'on utilise un acide nucléique peptidique en tant qu'analogue d'acide nucléique.

24. Procédé selon l'une des revendications 1 à 18 **caractérisé en ce que** l'on utilise un réactif de détection qui est en concurrence avec la substance à déterminer autour d'un site de liaison.

25. Procédé selon la revendication 24 **caractérisé en ce que** l'on utilise en tant que réactif de détection des anticorps ou des fragments d'anticorps lorsque la substance à déterminer est un anticorps.

26. Procédé selon la revendication 24 **caractérisé en ce que** l'on utilise en tant que réactif de détection des antigènes ou/et des haptènes lorsque la substance à déterminer est un antigène.

27. Procédé selon la revendication 24 **caractérisé en ce que** l'on utilise en tant que réactif de détection un oligomère ou un polymère d'acide nucléique ou un analogue d'acide nucléique lorsque la substance à déterminer est un acide nucléique.

28. Procédé selon la revendication 27 **caractérisé en ce que** l'on utilise un acide nucléique peptidique en tant qu'analogue d'acide nucléique.

29. Procédé selon l'une des revendications précédentes **caractérisé en ce que** le couplage de la streptavidine ou de l'avidine à la phase solide est réalisé à l'aide de sérum-albumine bovin thermique (SABT).

30. Procédé selon l'une des revendications précédentes **caractérisé en ce qu'**en tant que phase solide inactive on utilise un plateau à micro-titration dont les puits sont remplis de différents échantillons d'essai et **en ce que** ces différents échantillons d'essai sont transférés en tant que phase active sans retard de temps dans un autre plateau à micro-titration, de préférence au moyen d'une pipette à plusieurs canaux.

31. Procédé selon la revendication 30 **caractérisé en ce que** le réactif de captage et éventuellement aussi le réactif de détection sont présents lors de la pré-incubation avec la phase solide inactive.

32. Phase solide inactive destinée à être utilisée dans des immunoessais ou des essais d'hybridation d'acides nucléiques pour détecter qualitativement et/ou quantitativement une substance à déterminer dans un échantillon d'essai, pour laquelle on utilise les composants suivants :
a) un réactif de captage qui à l'aide de deux sites de liaison différents 1) le cas échéant conjointement avec d'autres composants d'essai permet de détecter spécifiquement la substance à déterminer et 2) est lié ou susceptible d'être lié à une phase solide active au moyen d'une paire de liaisons spécifique dont l'un des partenaires est lié au réactif de captage et dont l'autre partenaire est couplé à une phase solide active,
b) une phase solide active à laquelle est couplé le deuxième partenaire de la paire de liaisons spécifique,
**caractérisée en ce que** la phase solide inactive correspond à la phase solide active mais qu'elle est néanmoins modifiée de telle manière qu'il ne peut y avoir aucune liaison au réactif de captage.

33. Phase solide inactive selon la revendication 32 **caractérisée en ce qu'**elle présente en tant que phase solide un petit tube en plastique, un plateau à micro-titration, des perles en verre ou en plastique ou des particules en latex.

34. Phase solide inactive selon la revendication 32 ou 33 **caractérisée en ce qu'**elle comprend un partenaire d'une des paires de liaison biotine / streptavidine, biotine / avidine, antigène / anticorps, haptène / anticorps anti-haptène ou à chaque fois des fragments susceptibles de se lier les uns aux autres.

35. Phase solide inactive selon la revendication 34 **caractérisée en ce que** le partenaire de la paire de liaison est l'avidine ou la streptavidine.

36. Phase solide inactive selon la revendication 35 **caractérisée en ce que** le couplage de l'avidine ou de la streptavidine sur la phase solide est réalisé par recouvrement à l'aide de SAB thermique.

37. Phase solide inactive selon la revendication 35 ou 36 **caractérisée en ce que** l'inactivation est réalisée par saturation de l'avidine ou de la streptavidine à l'aide de biotine ou d'un dérivé de la biotine.

38. Phase solide inactive selon la revendication 35 ou 36 **caractérisée en ce que** l'inactivation est réalisée par une modification covalente du centre actif de l'avidine ou de la streptavidine.

39. Phase solide inactive selon la revendication 38 **caractérisée en ce que** pour la modification covalente on réalise une dérivation d'au moins un acide aminé du centre actif ou un couplage covalent de la biotine sur le centre actif.

40. Phase solide inactive selon la revendication 39 **caractérisée en ce que** le couplage covalent de la biotine sur le centre actif est réalisé par de la biotine photoactivable, par exemple de la biotine DADOO-AB.

41. Phase solide inactive selon la revendication 35 ou 36 **caractérisée en ce que** l'inactivation est réalisée par modification génétique du centre actif de l'avidine ou de la streptavidine comme la substitution, la délétion ou l'insertion d'un seul ou de plusieurs restes d'acide aminé.

42. Utilisation d'une phase solide inactive selon l'une des revendications 32 à 41 pour déparasiter des immunoessais ou des essais d'hybridation d'acides nucléiques par captage de composants à liaison non spécifique dans un échantillon d'essai à analyser.

43. Kit d'essai pour un immunoessai ou un essai d'hybridation d'acides nucléiques destiné à détecter qualitativement et/ou quantitativement une substance à déterminer dans un échantillon d'essai, laquelle contient les composants de l'immunoessai ou de l'essai d'hybridation d'acides nucléiques concerné ainsi qu'une phase solide inactive selon l'une des revendications 32 à 41.

44. Kit d'essai selon la revendication 43 **caractérisée en ce qu'**il comprend les composants suivants :
a) un réactif de captage qui à l'aide de deux sites de liaison différents 1) le cas échéant conjointement avec d'autres composants d'essai permet de détecter spécifiquement la substance à déterminer et 2) est lié ou susceptible d'être lié à une phase solide active au moyen d'une paire de liaisons spécifique dont l'un des partenaires est lié au réactif de captage et dont l'autre partenaire est couplé à une phase solide active,
b) un réactif de détection,
c) une phase solide active à laquelle est couplé le deuxième partenaire de la paire de liaisons spécifique et
d) une phase solide inactive qui correspond à la phase solide active mais qui est modifiée de telle sorte que le réactif de captage ne peut pas se lier.

45. Procédé pour éviter les perturbations par des liaisons non spécifiques à la phase solide dans un immunoessai ou dans un essai d'hybridation d'acides nucléiques par mise en oeuvre des composants suivants
a) un réactif de captage qui à l'aide de deux sites de liaison différents 1) le cas échéant conjointement avec d'autres composants d'essai permet de détecter spécifiquement la substance à déterminer et 2) est lié ou susceptible d'être lié à une phase solide active au moyen d'une paire de liaisons spécifique dont l'un des partenaires est lié au réactif de captage et dont l'autre partenaire est couplé à une phase solide active,
b) au moins deux phases solides actives,
**caractérisé en ce que**, avant d'ajouter le réactif de captage et éventuellement d'autres composants d'essai, on réalise une incubation de l'échantillon d'essai en présence d'une première phase solide active, l'échantillon d'essai étant ensuite séparé de cette première phase solide et une incubation étant ensuite réalisée en présence du réactif de captage et éventuellement d'autres composants d'essai et d'une deuxième phase solide pour l'essentiel identique, la substance à déterminer étant détectée de façon appropriée.
